Europäisches Patentamt

European Patent Office

Office européen des brevets

(11) Veröffentlichungsnummer: **0 219 447 B1**

(19)

## (12) EUROPÄISCHE PATENTSCHRIFT

(45) Veröffentlichungstag der Patentschrift: **01.09.93**

(51) Int. Cl.5: **A61K 31/565**

(21) Anmeldenummer: **86730120.2**

(22) Anmeldetag: **25.07.86**

(54) **Antigestagene zur Verschiebung der endometrialen Reifung.**

(30) Priorität: **13.09.85 DE 3533175**

(43) Veröffentlichungstag der Anmeldung:
**22.04.87 Patentblatt 87/17**

(45) Bekanntmachung des Hinweises auf die
Patenterteilung:
**01.09.93 Patentblatt 93/35**

(84) Benannte Vertragsstaaten:
**AT BE CH DE FR GB IT LI LU NL SE**

(56) Entgegenhaltungen:

**ACTA ENDOCRINOL., Band 111, suppl. 274,
1986; H.M.BEIER et al., Seiten 147-143***

**ADVANCES IN THE BIOSCIENCES, Band 6,
1971; H.M.BEIER et al., Seiten 165-189***

**PROG. REPROD. BIOL., Band 7, 1980, Basel
(CH); H.M.BEIER, Seiten 158-172***

**ARCH. GYNAECOL., Band 238, 1985;
H.M.BEIER, Seiten 67-71***

**EUR. J. BIOCHEM., Band 148, April 1985,
FEBS; M.RAUCH et al., Seiten 213-213***

**SERONO SYMP. PUBL., Band 35, 1987, Raven
Press; H.M.BEIER et al., Seiten 331-343***

**J. REPROD. FERT:, Band 35, 1973;
C:E:ADAMS, Seiten 613-614***

(73) Patentinhaber: **SCHERING AKTIENGESELL-
SCHAFT Berlin und Bergkamen
Müllerstrasse 170/178 Postfach 65 03 11
D-13303 Berlin(DE)**

(72) Erfinder: **Beier, Henning, Prof.Dr.Dr.
Rotbendenstr. 19
D-5100 Aachen(DE)**
Erfinder: **Elger, Walter, Dr.
Schorlemerallee 12b
D-1000 Berlin 33(DE)**

**Beschreibung**

Die Erfindung betrifft die Verwendung von Antigestagenen zur Herstellung eines Arzneimittels zur Verschiebung der endometrialen Reifung in der postovulatorischen Phase (Lutealphase) im Hinblick auf den Nidationszeitpunkt.

Ein in den letzten Jahren vor allem in der westlichen Welt stark wachsender Prozentsatz von Frauen sieht sich dem Problem der ungewollten Sterilität konfrontiert. Eine erstmals 1977/78 von den Briten Patrick Steptoe und Robert Edwards erfolgreich durchgeführte gynäkologisch-embryologische Technik weckte Hoffnung auf die Erfüllung ihres Kinderwunsches bei denjenigen Frauen, deren Sterilität durch eine auf keine Weise behebbaren Unpassierbarkeit der Eileiter hervorgerufen wird. Voraussetzung ist, daß wenigstens eines der Ovarien sowie der Uterus funktionsfähig sind.

Bei diesem Verfahren werden zunächst reife Eizellen laparoskopisch aus einem der Ovarien entnommen und anschließend in vitro befruchtet. Das nach dieser extrakorporalen Befruchtung in einem Kulturmedium herangewachsene Embryo (Zygote) wird dann nach ca. 48 bis 72 Stunden in den Uterus der Frau implantiert.

Bei diesem inzwischen weltweit häufig angewendeten Therapieverfahren der In-Vitro-Fertilisation und des Embryo-Transfers (IV F/ET) überleben jedoch aufgrund einer geringen Implantationserfolgsrate nur enttäuschend wenige menschliche Embryos. Die wesentliche Ursache hierfür ist ein verlangsamtes Wachstum der befruchteten Eizellen in der In-vitro-Kultur, so daß daraus eine Desynchronisation von Embryo und Endometrium resultiert.

Versuche mit Antigestagenen führten nun zu dem überraschenden Ergebnis, daß durch einen "Dämpfungseffekt" dieser Steroide auf das System "Corpus-luteum-Endometrium" in der post-ovulatorischen Phase eine Verzögerung für das Zustandekommen eines Präimplantationsendometriums bewirkt wird.

Dieser Effekt kann am erprobten Kaninchen/Uteroglobin-System gezeigt werden. Dieses hat im Vergleich zu allen anderen Spezies die Vorteile, daß die Lutealphase der menschlichen analog ist und daß Ovulation und das "Timing" der Präimplantationsphase exakt steuerbar sind. (Prog reprod Biol Vol. 7 Blastocyst-Endometrium Relationships. Karger, Basel, München, New York (1980) 158-172.)

Bei den Untersuchungen zeigte sich, daß es nach einer Behandlung mit einer wirksamen Antigestagen-Dosierung zu einer massiven Sekretionshemmung des progesteronabhängigen Proteins Uteroglobin kommt.

In den Tagen nach einer solchen Behandlung erholt sich jedoch überraschenderweise das System "Corpus-luteum-Endometrium" wieder, so daß man eine Uteroglobinsekretion beobachtet, wie sie früheren Präimplantationsphasen entspricht.

Durch diese Antigestagenbehandlung kann somit die für die Implantation der Blastocyste günstige Phase (rezeptive Phase des Endometriums) aktiv verschoben werden. Eine solche "Verschiebung" des Beginns der Lutealphase im Hinblick auf den Nidationszeitpunkt ist mit der Antigestagenbehandlung erstmals möglich, denn es gab bisher keine Therapie oder endokrinologische Maßnahme, durch Beeinflussung der post-ovulatorischen Phase einen möglichen optimalen Implantationszeitpunkt hinauszuschieben. Die durch Antigestagen-Applikation bewirkte Verzögerung der Endometriumsrezeptivität für die Blastocystenimplantation kommt damit einem zeitgerechten Retransfer der durch die In-vitro-Kultur verlangsamten Embryonalentwicklung entgegen.

Man erreicht somit durch die erfindungsgemäße Antigestagenbehandlung bei denjenigen Retransfers, die bisher keinerlei Chance auf eine erfolgreiche Implantation des Embryos hatten, eine hohe Erfolgsrate.

Nach einer bevorzugten Ausführungsform wird die Antigestagenbehandlung in Abhängigkeit von der gewünschten Verschiebung der endometrialen Reifung in der Lutealphase in der Regel über eine Dauer von 1 bis 6, vorzugsweise 1 bis 4 Tagen, beginnend mit der Ovulation bzw. der Entnahme von Oocyten vorgenommen.

Auch die pathologische Lutealphase (Short luteal phase, Defective luteal phase) kann mit einer Antigestagenbehandlung während der Zeit unmittelbar nach der Ovulation günstig beeinflußt werden. Damit kann eine hierdurch bedingte Sterilität mit der erfindungsgemäßen Verwendung von Antigestagenen behandelt werden.

Die Antigestagene werden gemäß vorliegender Erfindung in Mengen von im allgemeinen 10 - 200 mg 11$\beta$-[(4-N,N-Dimethylamino)-phenyl]-17$\beta$-hydroxy-17$\alpha$-(3-hydroxyprop-1-(Z)-enyl)-4,9(10)-estradien-3-on pro Tag oder einer biologisch äquivalenten Menge eines anderen Antigestagens eingesetzt.

Gemäß vorliegender Erfindung können Antigestagene auch in der veterinär-medizinischen Therapie vor allem bei landwirtschaftlichen Nutztieren, wie zum Beispiel Pferden, Rindern, Schweinen und Schafen, eingesetzt werden. Sie dienen dabei der Erzielung einer höheren Erfolgsrate bei der zum Beispiel aus ökonomischen Gründen oder Züchtungszwecken vorgenommenen In-vitro-Fertilisation und dem Embryo-Transfer.

Die Antigestagenbehandlung wird in der Regel über eine Dauer von 1 bis 6 Tagen, vorzugsweise 1 - 4 Tagen, beginnend mit der Ovulation bzw. der Entnahme von Oocyten, vorgenommen.

Die Antigestagene werden dabei in Mengen von im allgemeinen 0,1 bis 20 mg/kg/Tag $11\beta$-[(4-N,N-Dimethylamino)-phenyl]-$17\beta$-hydroxy-$17\alpha$-(3-hydroxyprop -1-(Z)-enyl)-4,9(10)-estradien-3-on oder einer biologisch äquivalenten Menge eines anderen Antigestagens eingesetzt.

Als verwendbare Antigestagene kommen alle Verbindungen infrage, die eine starke Affinität zum Gestagenrezeptor (Progesteronrezeptor) besitzen und dabei keine eigene gestagene Aktivität zeigen. Als kompetitive Progesteronantagonisten kommen beispielsweise folgende Steroide infrage:

$11\beta$-[(4-N,N-Dimethylamino)-phenyl]-$17\beta$-hydroxy-$17\alpha$-propinyl-4,9(10)-estradien-3-on,
$11\beta$-[(4-N,N-Dimethylamino)-phenyl]-$17\beta$-hydroxy-18-methyl-$17\alpha$-propinyl-4,9(10)-estradien-3-on und
$11\beta$-[(4-N,N-Dimethylamino)-phenyl]-17a$\beta$-hydroxy-17a$\alpha$-propinyl-D-homo-4,9(10)-16-estratrien-3-on
(Europäische Patentanmeldung 82400025.1 - Veröffentlichungsnummer 0 057 115),
$11\beta$-Methoxyphenyl-$17\beta$-hydroxy-$17\alpha$-ethinyl-4,9(10)-estradien-3-on (Steroids 37 (1981) 361 - 382),
$11\beta$-[(4-N,N-Dimethylamino)-phenyl]-$17\beta$-hydroxy-$17\alpha$-(hydroxyprop-1-(Z)-enyl)-4,9(10)-estradien-3-on
(Europäische Patentanmeldung 847300147.0 - Veröffentlichungsnummer 0 147 361),
$11\beta$-[(4-N,N-Dimethylamino)-phenyl]-$17\alpha$-hydroxy-$17\beta$-(3-hydroxypropyl)-13$\alpha$-methyl-4,9(10)-gonadien-3-on
(Europäische Patentanmeldung 84730062.1 - Veröffentlichungsnummer 0 129 499).

Die Antigestagene können zum Beispiel lokal, topisch, enteral oder parenteral appliziert werden.

Für die bevorzugte orale Applikation kommen insbesondere Tabletten, Dragées, Kapseln, Pillen, Suspensionen oder Lösungen infrage, die in üblicher Weise mit den in der Galenik gebräuchlichen Zusätzen und Trägersubstanzen hergestellt werden können. Für die lokale oder topische Anwendung kommen beispielsweise Vaginalzäpfchen oder transdermale Systeme wie Hautpflaster infrage.

Eine Dosiseinheit enthält etwa 2 bis 200 mg $11\beta$-[(4-N,N-Dimethylamino)-phenyl]-$17\beta$-hydroxy-$17\alpha$-(3-hydroxyprop-1(Z)-enyl)-4,9(10)-estradien-3-on oder eine biologisch äquivalente Menge eines anderen Antigestagens.

Die nachstehenden Beispiele sollen die galenische Formulierung von Antigestagenen erläutern.

Beispiel 1

Zusammensetzung einer Tablette mit 10 mg $11\beta$-[(4-N,N-Dimethylamino)-phenyl]-$17\beta$-hydroxy-$17\alpha$-(3-hydroxyprop -1-(Z)-enyl)-4,9(10)-estradien-3-on zur oralen Applikation

```
  10,0 mg   11ß-/(4-N,N-Dimethylamino)-phenyl/-17ß-
            hydroxy-17α-(3-hydroxyprop  -1-(Z)-enyl)-
            4,9(10)-estradien-3-on

 140,5 mg   Laktose

  69,5 mg   Maisstärke

   2,5 mg   Polyvinylpyrrolidon 25

   2,0 mg   Aerosil

   0,5 mg   Magnesiumstearat

 225,0 mg   Gesamtgewicht
 ========
```

Beispiel 2

Zusammensetzung einer öligen Lösung mit 50 mg $11\beta$-[(4-N,N-Dimethylamino)-phenyl]-$17\beta$-hydroxy-$17\alpha$-(3-hydroxyprop -1-(Z)-enyl)-4,9(10)-estradien-3-on zur parenteralen Applikation

In je 1 ml Rizinusöl/Benzylbenzoat im Volumenverhältnis 6 : 4 werden 50 mg des Antigestagens gelöst.

Pharmakologische Beobachtungen

Versuche:

Mit der Uterusproteinsekretion des Kaninchenendometriums steht ein sicheres Modell zur Verfügung, Progesteroneffekte zu beurteilen. Die Synthese und Sekretion des Uteroglobins ist in diesem Modell nachweislich Progesteron-abhängig. Damit hat man einen für Progesteron hochspezifischen Marker, der in elektrophoretischen Analysensystemen sicher interpretiert werden kann.

Dieses Modell mit den Progesteron-abhängigen Proteinen und dem Markerprotein Uteroglobin ist geeignet, Wirkungen von Antigestagenen zu prüfen und zu beurteilen.

In einer zeitabhängigen Studie wurde das Antigestagen 11$\beta$-[(4-N,N-Dimethylamino)-phenyl]-17$\beta$-hydroxy-17$\alpha$-(3-hydroxyprop -1-(Z)-enyl)-4,9(10)-estradien-3-on auf seine Wirkung geprüft, einerseits auf die Sofortwirkung nach Behandlung, andererseits auf die Langzeitwirkung nach Ende der Behandlung.

Kaninchen wurden mit einer hCG-Injektion (150 I.E. i.v.) in den Zustand der Pseudogravidität versetzt (Tag 0). Am Tag 2, Tag 3 und Tag 4 erhielten die Tiere per Magensonde eine Antigestagenbehandlung von 20 mg/pro kg Körpergewicht/pro Tag. Am Tag 5, also 24 Stunden nach Abschluß der Behandlung, wurde das Uterusproteinmuster elektrophoretisch analysiert.

Ergebnisse:

Uteroglobin verschwindet im Vergleich zu unbehandelten Kontrollen und Kontrolltieren, welche nur 1/10 der Dosis erhielten, fast völlig aus dem Sekret. Das veränderte Proteinmuster entspricht einer Hemmung der Corpus-luteum-Funktion.

Wenn die Uteroglobinsekretion und das übrige Proteinmuster zu späteren Zeitpunkten nach Abschluß der Behandlung analysiert werden, so ergibt sich überraschenderweise eine deutliche "Erholung" der Progesteron-Hemmung bzw. eine zunehmend stärkere Progesteronwirkung. Man sieht 48 Stunden, 72 Stunden sowie 96 Stunden nach Absetzen des Antigestagens, daß Uteroglobin in einer physiologischen Weise sezerniert wird, wie sie sonst früheren Präimplantationsphasen entspricht. Hieraus folgt, daß das Antigestagen am Kaninchen-Corpus-luteum und/oder am Endometrium eine Progesteron-Hemmung bewirkt, die als "Dämpfung" oder reversible Hemmung bezeichnet werden kann.

**Patentansprüche**

1. Verwendung von Antigestagenen (AG) zur Herstellung eines Arzneimittels zur Verschiebung (Verzögerung) der endometrialen Reifung in der post-ovulatorischen Phase (Lutealphase) im Hinblick auf den Nidationszeitpunkt.

2. Verwendung von 11$\beta$-[(4-N,N-Dimethylamino)-phenyl]-17$\beta$-hydroxy-17$\alpha$-propinyl-4,9(10)-estradien-3-on, 11$\beta$-[(4-N,N-Dimethylamino)-phenyl]-17$\beta$-hydroxy-18-methyl-17$\alpha$-propinyl-4,9(10)- estradien-3-on, 11$\beta$-[(4-N,N-Dimethylamino)-phenyl]-17a$\beta$-hydroxy-17a$\alpha$-propinyl-D-homo-4,9(10)-16-estratrien-3-on, 11$\beta$-Methoxyphenyl-17$\beta$-hydroxy-17$\alpha$-ethinyl-4,9(10)-estradien-3-on, 11$\beta$-[(4-N,N-Dimethylamino)-phenyl]-17$\beta$-hydroxy-17$\alpha$-(3-hydroxyprop-1-(Z)-enyl)-4,9(10)-estradien-3-on, 11$\beta$-[(4,N,N-Dimethylamino)-phenyl]-17$\alpha$-hydroxy-17$\beta$-(3-hydroxypropyl)-13$\alpha$-methyl-4,9(10)-gonadien-3-on, als Antigestagen gemäß Anspruch 1.

**Claims**

1. The use of antigestagens (AG) for the preparation of a medicament for shifting (delaying) endometrial maturation in the post-ovulatory phase (luteal phase) with respect to the time of implantation.

2. The use of 11$\beta$-[4-(N,N-dimethylamino)-phenyl]-17$\beta$-hydroxy-17$\alpha$-propynyl-4,9(10)-oestradien-3-one, 11$\beta$-[4-(N,N-dimethylamino)-phenyl]-17$\beta$-hydroxy-18-methyl-17$\alpha$-propynyl-4,9(10)-oestradien-3-one, 11$\beta$-[4-(N,N-dimethylamino)-phenyl]-17a$\beta$-hydroxy-17a$\alpha$-propynyl-D-homo-4,9(10)-16-oestratrien-3-one, 11$\beta$-methoxyphenyl-17$\beta$-hydroxy-17$\alpha$-ethynyl-4,9(10)-oestradien-3-one, 11$\beta$-[4-(N,N-dimethylamino)-phenyl]-17$\beta$-hydroxy-17$\alpha$-(3-hydroxyprop-1-(Z)-enyl)-4,9(10)-oestradien-3-one,

EP 0 219 447 B1

11$\beta$-[4-(N,N-dimethylamino)-phenyl]-17$\alpha$-hydroxy-17$\beta$-(3-hydroxypropyl)-13$\alpha$-methyl-4,9(10)-gonadien-3-one,

as antigestagen according to claim 1.

**Revendications**

1. Utilisation d'antigestagènes (AG) pour la préparation d'un médicament pour le décalage (le retard) de la maturation de l'endomètre dans la phase post-ovulation (phase lutéale) par rapport au moment de la nidation.

2. Utilisation de :
la 11$\beta$-[4-N,N-diméthylamino)-phényl]-17$\beta$-hydroxy-17$\alpha$-propynyl-4,9(10)-oestradiène-3-one,
la 11$\beta$-[(4-N,N-diméthylamino)-phényl]-17$\beta$-hydroxy-1$\beta$-méthyl-17$\alpha$-propynyl-4,9-(10)-oestradiène-3-one,
la 11$\beta$-[(4-N,N-diméthylamino)-phényl]-17a$\beta$-hydroxy-17a$\alpha$-propynyl-D-homo-4,9-(10)-16-oestratriène-3-one,
la 11$\beta$-méthoxyphényl-17$\beta$-hydroxy-17$\alpha$-éthynyl-4,9(10)-oetradiène-3-one,
la 11$\beta$-[(4-N,N-diméthylamino)-phényl]-17$\beta$-hydroxy-17$\alpha$-(3-hydroxyprop-1-(Z)-ényl)-4,9(10)oestradiène-3-one,
la 11$\beta$-[(4-N,N-diméthylamino)-phényl]-17$\alpha$-hydroxy-17$\beta$-(3-hydroxypropyl)-13$\alpha$-méthyl-4,9(10)-gonadiène-3-one,

comme anti-gestagènes selon la revendication 1.

5